# EUROPEAN PATENT APPLICATION

(11) **EP 4 159 183 A1**
(43) Date of publication of application: **05.04.2023**
(21) Application number: 21818543.7
(22) Date of filing: 27.05.2021
(51) Int. Cl.: A61H 21/00

(54) **CAPSULE APPARATUS AND STARTING METHOD THEREFOR**

(30) Priority: 01.06.2020 CN 202010486164
(71) Applicant: Ankon Medical Technologies (Shanghai) Co., Ltd, Shanghai 200131 (CN)
(72) Inventor: DUAN, Xiaodong, Shanghai 201206 (CN); DU, Jianming, Shanghai 201206 (CN)
(74) Representative: Lavoix
(86) International application number: PCT/CN2021/096442
(87) International publication number: WO 2021/244396

(57) **Abstract**

A capsule apparatus comprising: an enclosure and a capsule core arranged in the enclosure. The capsule core comprises: a power supply, an acceleration sensor connected to the power supply, a switching element having an input terminal connected to the power supply and a control terminal connected to the acceleration sensor; a control unit connected to a first output terminal of the switching element; and a working system connected to a second output terminal of the switching element. The capsule core also comprises a single-chip microcomputer, the control unit is integrated in the single-chip microcomputer, and the output terminal of the switching element is connected to the single-chip microcomputer. The first output terminal and the second output terminal of the switching element are respectively connected to the control unit and the working system, so that the acceleration sensor can control the power on and off of the control unit and the working system according to different states, so that the working state of the capsule apparatus can be changed only by the acceleration sensor without the assistance of external equipment, which makes the use more convenient.

## Description

### CROSS-REFERENCE TO RELATED APPLICATION

The application claims priority from Chinese Patent Application No. 202010486164.5, filed Jun 1, 2020, entitled "capsule apparatus and starting method therefor", all of which are incorporated herein by reference in their entireties.

### TECHNICAL FIELD

The present invention relates to a technique for a swallowable device, and more particularly to a capsule apparatus and a starting method therefor.

### BACKGROUND

Currently, capsule apparatuses are widely used in medical practice. Among them, capsule endoscopy has achieved great results in the examination of patients in vivo and has shown obvious advantages over traditional endoscope in the examination of small intestine of patients, which can detect intestinal regions and parts that cannot be detected by traditional endoscope. A vibrating capsule acts on the colon wall of a human body through vibration, can effectively relieve colon spasm, promote colon movement, treat constipation, and promote defecation, thereby achieving the effects of maintaining beauty, keeping young and preserving health. Moreover, the vibrating capsule can promote the peristalsis of the small intestine and reduce the absorption of food by the small intestine, thus achieving the effect of losing weight.

However, in the existing capsule apparatus, due to its independence, integrity and tightness of the enclosure, the method of starting the capsule apparatus is usually through a noncontact switch, such as a photoelectric switch, a magnetic switch, etc. All of these switches require a help of specified auxiliary tools outside the capsule apparatus to work properly and are susceptible to environmental influences that can easily put the capsule apparatus into abnormal operating conditions.

Therefore, it is necessary to design a capsule apparatus that does not require an external auxiliary tool for starting and a starting method thereof.

### SUMMARY

It is one object of the present invention to provide a capsule apparatus comprising: an enclosure and a capsule core arranged in the enclosure. The capsule core comprises a power supply, an acceleration sensor connected to the power supply, a switching element comprising an input terminal connected to the power supply and a control terminal connected to the acceleration sensor, a control unit connected to the first output terminal of the switching element, and a working system connected to the second output terminal of the switching element. The capsule core further comprises a single-chip microcomputer, the control unit is integrated in the single-chip microcomputer, and the output terminal of the switching element is connected to the single-chip microcomputer.

In one embodiment, the switching element comprises a first MOS transistor and a second MOS transistor, a first output terminal of the first MOS transistor is connected to the control unit through the single-chip microcomputer, and a second output terminal of the second MOS transistor is connected to the working system.

It is another object of the present invention to provide a capsule apparatus comprising: an enclosure and a capsule core arranged in the enclosure. The capsule core comprises a power supply, an acceleration sensor connected to the power supply, a switching element comprising an input terminal connected to the power supply and a control terminal connected to the acceleration sensor, a control unit connected to the first output terminal of the switching element, and a working system connected to the second output terminal of the switching element. The capsule core further comprises a single-chip microcomputer, the control unit, the acceleration sensor, and the switching element are integrated in the single-chip microcomputer, and the output terminal of the switching element extends to the outside of the single-chip microcomputer and is connected to the working system.

In one embodiment, the switching element comprises a first MOS transistor and a second MOS transistor, a first output terminal of the first MOS transistor is connected to the control unit, and a second output terminal of the second MOS transistor is connected to the working system.

It is still another object of the present invention to provide a starting method of the capsule apparatus, the starting method comprising: receiving an acceleration value detected by the acceleration sensor; determining whether the detected acceleration value reaches a preset value; driving the power supply to supply power to the control unit inside the capsule apparatus when the detected acceleration value reaches the preset value; detecting a specific operation of the capsule apparatus and identifying a gesture applied to the capsule apparatus; determining whether the gesture is a preset action; and driving the power supply to supply power to the working system inside the capsule apparatus when the gesture is the preset action.

In one embodiment, before the step "the detected acceleration value reaches the preset value" comprises: controlling the power supply to power on the acceleration sensor; controlling the acceleration sensor to enter a slow detection mode; controlling the first MOS transistor to be disconnected to power off the control unit by the acceleration sensor, and controlling the second MOS transistor to be disconnected to power off the working system by the acceleration sensor.

In one embodiment, after the step "the detected acceleration value reaches the preset value" comprises: controlling the acceleration sensor to enter a fast detection mode.

In one embodiment, the step "driving the power supply to supply power to the control unit inside the capsule apparatus" specifically comprises: controlling the first MOS transistor to be connected to enable the power supply to supply power to the control unit by the acceleration sensor.

In one embodiment, after the step "the detected acceleration value reaches the preset value" comprises: controlling the acceleration sensor to enter a gesture recognition mode, and the step "detecting the specific operation of the capsule apparatus" is specifically comprises: controlling the acceleration sensor to continuously detect the acceleration value so as to determine a specific operation of the capsule apparatus.

In one embodiment, the step "driving the power supply to supply power to the working system inside the capsule apparatus" specifically comprises: controlling the second MOS transistor to be connected to power on the working system by the acceleration sensor.

Compared with the prior art, the first output terminal and the second output terminal of the switching element are respectively connected to the control unit and the working system, so that the acceleration sensor can control the power on and off of the control unit and the working system according to different states, so that the working state of the capsule apparatus can be changed only by the acceleration sensor without the assistance of external equipment, which makes the use more convenient.

### BRIEF DESCRIPTION OF THE DRAWINGS

FIG. 1 illustrates a process flow diagram of a starting method of a capsule apparatus according to the present invention.
FIG. 2 is a partial structure diagram of the capsule apparatus according to a first embodiment of the present invention.
FIG. 3 is a partial structure diagram of the capsule apparatus according to a second embodiment of the present invention.

### DETAILED DESCRIPTION

In order to enable those in the art to better understand technical solutions in the present invention, the technical solutions in the embodiments of the present invention will be clearly and completely described below in conjunction with accompanying drawings in the embodiments of the present invention. It is clear that the embodiments described are only a part of the embodiments of the present invention, and the present invention is capable of other embodiments or of being practiced or carried out in various ways. Based on the embodiments in the present invention, all other embodiments obtained by a person of ordinary skill in the art without making creative labor shall fall within the scope of protection of the present invention.

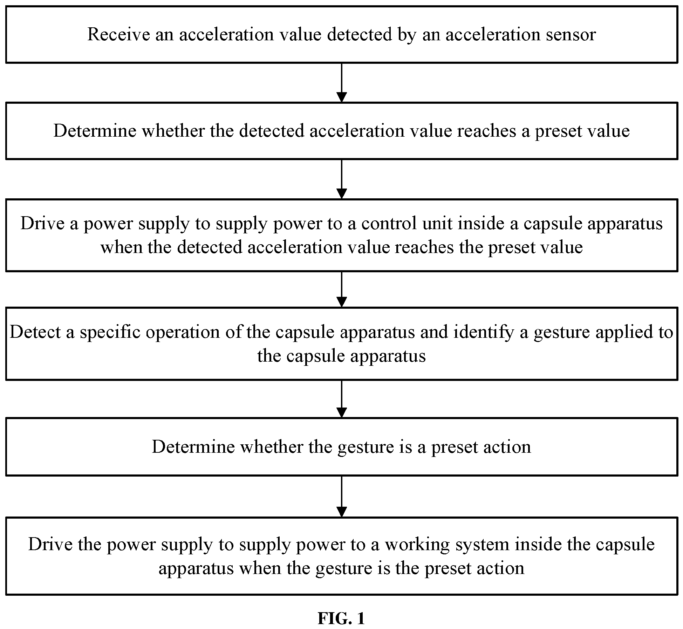

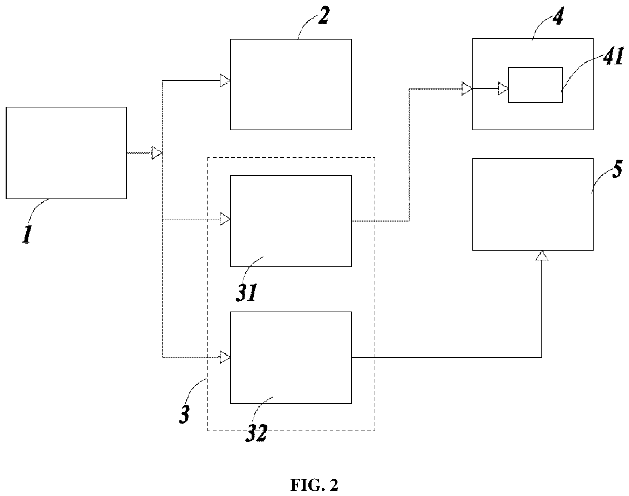

Referring to FIGS. 1-3, the present invention provides a capsule apparatus and a starting method therefor. Specifically, as shown in FIG. 2, in a first embodiment of the present invention, a capsule apparatus is provided, which comprises an enclosure and a capsule core arranged in the enclosure, wherein the capsule core comprises:
a power supply 1;
an acceleration sensor 2 connected to the power supply 1;
a switching element 3 comprising an input terminal connected to the power supply 1 and a control terminal connected to the acceleration sensor 2;
a control unit 41 connected to a first output terminal of the switching element 3; and
a working system 5 connected to a second output terminal of the switching element 3.

The capsule core further comprises a single-chip microcomputer 4, the control unit 41 is integrated in the single-chip microcomputer 4, and the output terminal of the switching element 3 is connected to the single-chip microcomputer 4.

In this embodiment, the control unit 41 is integrated in the single-chip microcomputer 4, and the output terminal of the switching element 3 is not directly communicated with the control unit 41, but the power supply 1 enters the single-chip microcomputer 4 through the switching element 3 first, and then is communicated with the control unit 41. The working system 5, such as a vibration motor and a camera, etc., is disposed outside the single-chip microcomputer 4.

The first output terminal of the switching element 3 and the second output terminal of the switching element 3 are respectively connected to the control unit 41 and the working system 5, so that the acceleration sensor 2 can control the power on and off of the control unit 41 and the working system 5 according to different states, so that the working state of the capsule apparatus can be changed only by the acceleration sensor 2 without the assistance of external equipment, which makes the use more convenient.

The switching element 3 comprises a first MOS transistor (metal oxide semiconductor field effect transistors) 31 and a second MOS transistor 32. A first output terminal of the first MOS transistor 31 is connected to the control unit 41 through the single-chip microcomputer 4, and a second output terminal of the second MOS transistor 32 is connected to the working system 5. The first MOS transistor 31 and the second MOS transistor 32 are both field effect transistors, and can receive switching signals of the acceleration sensor 2 to switch on or off the first MOS transistor 31 or the second MOS transistor 32, thereby affecting the switching on/off between the control unit 41 and the working system 5.

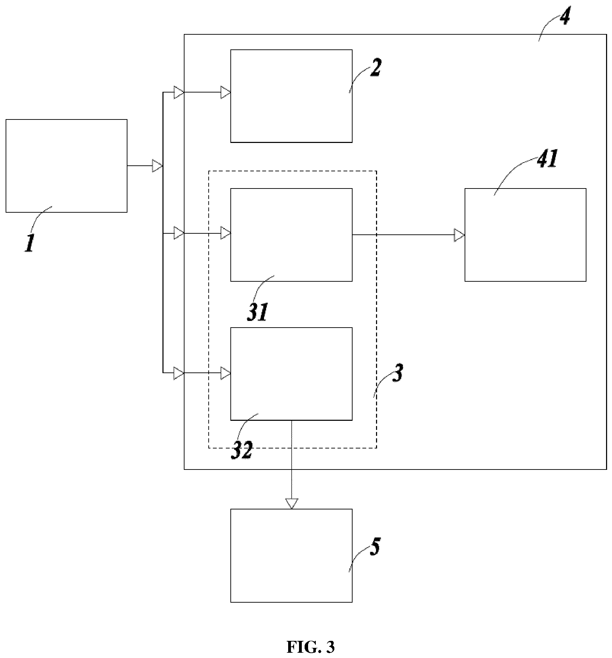

Further, as shown in FIG. 3, in a second embodiment of the present invention, a capsule apparatus is provided, which comprises: an enclosure and a capsule core arranged in the enclosure. The capsule core comprises:
a power supply 1;
an acceleration sensor 2 connected to the power supply 1;
a switching element 3 comprising an input terminal connected to the power supply 1 and a control terminal connected to the acceleration sensor 2;
a control unit 41 connected to a first output terminal of the switching element 3; and
a working system 5 connected to a second output terminal of the switching element 3.

The capsule core further comprises a single-chip microcomputer 4, wherein the control unit 41, the acceleration sensor 2 and the switching element 3 are integrated in the single-chip microcomputer 4, and the output terminal of the switching element 3 extends to the outside of the single-chip microcomputer 4 and is connected to the working system 5.

Different from the first embodiment, the control unit 41, the acceleration sensor 2 and the switching element 3 are all integrated in the single-chip microcomputer 4, such that the power supply 1 must first enter the single-chip microcomputer 4 and then be connected to the acceleration sensor 2 and the switching element 3. The switching element 3 can control the power on and off of the control unit 41 which is also integrated in the single-chip microcomputer 4, and the working system 5 such as a vibration motor and a camera, etc., is arranged outside the single-chip microcomputer 4, and the output terminal of the switching element 3 passes through the single-chip microcomputer 4 and is in communication with the working system 5.

Similarly, the first output terminal of the switching element 3 and the second output terminal of the switching element 3 are respectively connected to the control unit 41 and the working system 5, so that the acceleration sensor 2 can control the power on and off of the control unit 41 and the working system 5 according to different states, so that the working state of the capsule apparatus can be changed only by the acceleration sensor 2 without the assistance of external equipment, which makes the use more convenient.

The switching element 3 comprises a first MOS transistor 31 and a second MOS transistor 32. A first output terminal of the first MOS transistor 31 is connected to the control unit 41, and a second output terminal of the second MOS transistor 32 is connected to the working system 5. The first MOS transistor 31 and the second MOS transistor 32 are both field effect transistors, and can receive switching signals of the acceleration sensor 2 to switch on or off the first MOS transistor 31 or the second MOS transistor 32, thereby affecting the switching on/off between the control unit 41 and the working system 5.

To sum up, the present invention provides two types of capsule apparatus, and the structure inside the enclosure of the capsule apparatus is arranged through different settings of the single-chip microcomputer 4.

Referring to FIG. 1, the present invention provides a starting method of a capsule apparatus, which comprises:
receiving an acceleration value detected by the acceleration sensor 2;
determining whether the detected acceleration value reaches a preset value;
driving the power supply 1 to supply power to the control unit 41 inside the capsule apparatus when the detected acceleration value reaches the preset value;
detecting a specific operation of the capsule apparatus and identifying a gesture applied to the capsule apparatus;
determining whether the gesture is a preset action; and
driving the power supply 1 to supply power to the working system 5 inside the capsule apparatus when the gesture is the preset action.

Therefore, in order to make the capsule apparatus work, two steps are comprised: first, detecting the acceleration value by the acceleration sensor 2 to drive the power supply to power on the control unit 41, and then identifying the gesture to drive the power supply to power on the working system 5, so that external auxiliary equipment is not needed to start the capsule apparatus, and the cost of the capsule apparatus can be reduced. In addition, the capsule apparatus can be started by gestures such as overturning, swinging, and shaking, etc., which enhances the interactivity between the capsule apparatus and a user, and better reflects the intellectualization and humanization of the capsule apparatus. Moreover, the preset value is a fixed acceleration value obtained through experiments, and the range is approximately between 1.0 g and 3.0 g, and g is the acceleration of gravity.

Before the step "the detected acceleration value reaches the preset value" comprises:
controlling the power supply 1 to power on the acceleration sensor 2;
controlling the acceleration sensor 2 to enter a slow detection mode;
controlling the first MOS transistor 31 to be disconnected to power off the control unit 41 by the acceleration sensor 2, and controlling the second MOS transistor 32 to be disconnected to power off the working system 5 by the acceleration sensor 2.

That is, before the detected acceleration value reaches the preset value, the acceleration sensor 2 is connected to the power supply 1 and powered on continuously, so that the acceleration value can be detected. However, at this time, the acceleration sensor 2 is in the slow detection mode, that is, the detection frequency of the acceleration sensor 2 is set to a first detection frequency, and the first detection frequency is relatively low. In this state, the control unit 41 and the working system 5 are powered off due to the disconnection of the first MOS transistor 31 and the second MOS transistor 32, and the entire capsule apparatus is in a low power consumption state.

When the acceleration value detected by the acceleration sensor 2 reaches the preset value, the acceleration sensor 2 enters a fast detection mode. In the fast detection mode, the detection frequency is set to a second detection frequency, and the second detection frequency is much greater than the first detection frequency, so that the specific operation of the capsule apparatus can be detected more accurately, and the gesture applied to the capsule apparatus can be identified.

Further, the step "driving the power supply 1 to supply power to the control unit 41 inside the capsule apparatus" specifically comprises:
controlling the first MOS transistor 31 to be connected to enable the power supply 1 to supply power to the control unit 41 by the acceleration sensor 2.

The control unit 41 is connected to the first MOS transistor 31. When the first MOS transistor 31 is closed, the control unit 41 is directly connected to the power supply 1, so that the control unit 41 is powered on. When the control unit 41 is powered on, it can cooperate with the acceleration sensor 2, and then carry out operations such as calculation and control.

Further, after the step "the detected acceleration value reaches the preset value" comprises:
controlling the acceleration sensor 2 to enter a gesture recognition mode.

The step "detecting the specific operation of the capsule apparatus" specifically comprises:
controlling the acceleration sensor 2 to continuously detect the acceleration value so as to determine the specific operation of the capsule apparatus.

In the present invention, the detection and recognition are also completed by the acceleration sensor 2 or the acceleration sensor 2 in cooperation with the control unit 41.

Specifically, after the acceleration value detected by the acceleration sensor 2 reaches the preset value, the acceleration sensor 2 not only enters the fast detection mode, but also enters the gesture recognition mode. The acceleration sensor 2 determines the specific operation of the capsule apparatus by detecting the change of the acceleration value within a period of time, and further recognizes the gesture applied to the capsule apparatus according to the gesture recognition algorithm. The recognized gesture is compared with a preset action, and if the gesture is the preset action, the working system 5 is driven to be powered on.

Furthermore, the step "driving the power supply 1 to supply power to the working system 5 inside the capsule apparatus" specifically comprises:
controlling the second MOS transistor 32 to be connected to power on the working system 5 by the acceleration sensor 2.

Therefore, in summary, the present invention provides a starting method of a capsule apparatus, wherein the capsule apparatus starts to work by detecting an acceleration value by the acceleration sensor 2 and recognizing a gesture applied to the capsule apparatus. Therefore, the external auxiliary equipment of the capsule apparatus can be minimized and the cost of the capsule apparatus can be reduced.

Furthermore, it should be understood that although the specification is described according to embodiments, not each embodiment contains only one separate technical solution, and that the specification is described in this manner only for clarity, and that those skilled in the art should consider the specification as a whole, and that the technical solutions in each embodiment may be suitably combined to form other embodiments as may be understood by those skilled in the art.

The series of detailed descriptions listed above are only specific to the feasible embodiments of the present invention and are not intended to limit the scope of protection of the present invention, and any equivalent embodiments or changes made without departing from the spirit of the art of the present invention shall be included in the scope of protection of the present invention.

## Claims

1. A capsule apparatus, comprising: an enclosure and a capsule core arranged in the enclosure, wherein the capsule core comprises:
a power supply;
an acceleration sensor connected to the power supply;
a switching element comprising an input terminal connected to the power supply and a control terminal connected to the acceleration sensor;
a control unit connected to a first output terminal of the switching element;
a working system connected to a second output terminal of the switching element; and
wherein the capsule core further comprises a single-chip microcomputer, wherein the control unit is integrated in the single-chip microcomputer, and the output terminal of the switching element is connected to the single-chip microcomputer.

2. The capsule apparatus of claim 1, wherein the switching element comprises a first MOS transistor and a second MOS transistor, wherein a first output terminal of the first MOS transistor is connected to the control unit through the single-chip microcomputer, and a second output terminal of the second MOS transistor is connected to the working system.

3. A capsule apparatus, comprising: an enclosure and a capsule core arranged in the enclosure, wherein the capsule core comprises:
a power supply;
an acceleration sensor connected to the power supply;
a switching element comprising an input terminal connected to the power supply and a control terminal connected to the acceleration sensor;
a control unit connected to a first output terminal of the switching element;
a working system connected to a second output terminal of the switching element; and
wherein the capsule core further comprises a single-chip microcomputer, wherein the control unit, the acceleration sensor and the switching element are integrated in the single-chip microcomputer, and the output terminal of the switching element extends to outside of the single-chip microcomputer and is connected to the working system.

4. The capsule apparatus of claim 3, wherein the switching element comprises a first MOS transistor and a second MOS transistor, wherein a first output terminal of the first MOS transistor is connected to the control unit, and a second output terminal of the second MOS transistor is connected to the working system.

5. A starting method of the capsule apparatus of any of claims 1-4, comprises:
receiving an acceleration value detected by the acceleration sensor;
determining whether the detected acceleration value reaches a preset value;
driving the power supply to supply power to the control unit inside the capsule apparatus when the detected acceleration value reaches the preset value;
detecting a specific operation of the capsule apparatus and identifying a gesture applied to the capsule apparatus;
determining whether the gesture is a preset action; and
driving the power supply to supply power to the working system inside the capsule apparatus when the gesture is the preset action.

6. The starting method of claim 5, wherein before the step of "the detected acceleration value reaches the preset value" comprises:
controlling the power supply to power on the acceleration sensor;
controlling the acceleration sensor to enter a slow detection mode;
controlling a first MOS transistor of the switching element to be disconnected to power off the control unit by the acceleration sensor, and controlling a second MOS transistor of the switching element to be disconnected to power off the working system by the acceleration sensor.

7. The starting method of claim 5, wherein after the step of "the detected acceleration value reaches the preset value" comprises:
controlling the acceleration sensor to enter a fast detection mode.

8. The starting method of claim 5, wherein the step of "driving the power supply to supply power to the control unit inside the capsule apparatus" specifically comprises:
controlling a first MOS transistor of the switching element to be connected to enable the power supply to supply power to the control unit by the acceleration sensor.

9. The starting method of claim 5, wherein after the step of "the detected acceleration value reaches the preset value" comprises:
controlling the acceleration sensor to enter a gesture recognition mode;
wherein the step of "detecting a specific operation of the capsule apparatus" comprises:
controlling the acceleration sensor to continuously detect the acceleration value to determine the specific operation of the capsule apparatus.

10. The starting method of claim 5, wherein the step of "driving the power supply to supply power to the working system inside the capsule apparatus" comprises:
controlling a second MOS transistor of the switching element to be connected to power on the working system by the acceleration sensor.
